# EUROPEAN PATENT APPLICATION

(11) **EP 4 410 204 A1**
(43) Date of publication of application: **07.08.2024**
(21) Application number: 24154729.8
(22) Date of filing: 30.01.2024
(51) Int. Cl.: A61B 5/16, A61B 5/18

(54) **SYSTEM AND METHOD FOR INDIVIDUALIZED MISSION RISK ASSESSMENT BASED ON PILOT PHYSIOLOGICAL MONITORING**

(30) Priority: 01.02.2023 US 202318104528
(71) Applicant: Rockwell Collins, Inc., Cedar Rapids, IA 52498 (US)
(72) Inventor: MATTHEWS, Cheyenne B., Marion, IA, 52302 (US); JOHNSON, Kevin, Cedar Rapids, IA, 52404 (US)
(74) Representative: Dehns

(57) **Abstract**

A system for assessing safe fulfillment of a mission plan or flight plan by a pilot receives physiological pilot monitoring data sensed inflight while the pilot is executing a flight plan. The system correlates pilot monitoring data with specific flight operations performs a post-flight fatigue assessment of the pilot (e.g., a performance score with respect to the completed flight plan and/or an assessment of the current fatigue state of the pilot). The system adds the pilot's post-flight fatigue state assessment to an individualized pilot profile. On receiving a subsequent flight plan for fulfillment by the pilot, the system performs a pre-flight risk assessment based on all available information, e.g., a risk assessment with respect to the pilot's fulfillment of the flight plan as a whole and/or specific risk assessments corresponding to fulfillment of component flight operations of the flight plan.

## Description

### BACKGROUND

Commanders develop fighter management plans to optimize assets available at a particular location or to a particular unit (e.g., aircraft, pilots, crew) and ensure 24-hour continuous operations at that location. Each fighter unit, for example, establishes a unique fighter management cycle for their assigned flight operations (e.g., instrument meteorological conditions (IMC), instrument flight rules (IFR), night-vision goggles (NVG), high-temperature operations, nighttime operations). Each pilot or crewmember, for example, may be assigned a 16-hour duty day and will maintain a constant state of rest with the goal of acquiring 8 continuous hours of rest, at which point the duty day resets. Pilots-in-command (PIC) are responsible for monitoring duty days and rest periods while on duty.

However, each individual mission of a particular duration, and each component flight operation of the mission, may affect different crewmembers in different ways with respect to cognitive workloads associated with different flight operations and crewmember fatigue levels during, and after, fulfillment of the mission, and including a particular combination of flight operations. Conventional duty day restrictions and rest period requirements do not account for individual tolerances for fatigue and/or cognitive workload, and the pilots and crewmembers themselves may not be reliably accurate judges of their own tolerance levels.

### SUMMARY

In a first aspect, a system for assessing safe fulfillment of a mission plan or flight plan by a pilot is disclosed. In embodiments, the system includes memory or data storage for storing a flight plan comprising a set or sequence of flight operations. The system includes processors for receiving physiological pilot monitoring data sensed inflight while the pilot is executing a flight plan (e.g., either inflight or after the flight has concluded). Based on the sensed physiological monitoring data, the system performs fatigue observations, e.g., by correlating (either inflight or postflight) pilot monitoring data with specific flight operations executed in fulfillment of the flight plan. Based on the sensed monitoring data and fatigue observations drawn therefrom, the system performs a post-flight fatigue assessment of the pilot (e.g., a performance score with respect to the completed flight plan and/or an assessment of the current fatigue state of the pilot). The system adds the pilot's post-flight fatigue state assessment to an individualized pilot profile. On receiving a subsequent flight plan for fulfillment by the pilot, the system performs a pre-flight risk assessment based on all available information, e.g., a risk assessment with respect to the pilot's fulfillment of the flight plan as a whole and/or specific risk assessments corresponding to fulfillment of component flight operations of the flight plan.

In some embodiments, the pilot monitoring data includes eye tracking data.

In some embodiments, the pilot monitoring data is captured by cockpit-based physiological sensors.

In some embodiments, the pre-flight risk assessment includes an authority level required for approval of the subsequent flight plan for execution by the pilot.

In some embodiments, the pilot profile includes an individualized baseline performance level. Further, the pilot profile may include indicators of the effect of a flight plan duration on baseline performance, or of the effect of particular flight operations on baseline performance.

In some embodiments, the post-flight fatigue assessment includes an individualized rest period recommendation based on the pilot's assessed fatigue state.

In a further aspect, a computer-assisted method for assessing fulfillment of a flight plan/mission plan by a pilot is also disclosed. In embodiments, the method includes receiving physiological pilot monitoring data sensed inflight while the pilot is executing a flight plan (e.g., a set of sequence of component flight operations for fulfillment through a duration). The method includes performing inflight fatigue observations by correlating the sensed monitoring data with concurrent flight operations. The method includes performing a post-flight fatigue assessment based on the sensed monitoring data and inflight fatigue observations, the post-flight assessment assigning the pilot a performance score for the completed flight plan and assessing the current fatigue state of the pilot. The method includes adding the inflight observations and post-flight assessment to the pilot's individualized profile. The method includes receiving a subsequent flight plan for execution by the pilot. The method includes performing a pre-flight risk assessment including a risk assessment associated with fulfillment of the subsequent flight plan by the pilot and/or a risk assessment associated with the fulfillment of one or more component flight operations of the subsequent flight plan by the pilot.

In some embodiments, the method includes receiving pilot eye tracking data sensed inflight.

In some embodiments, the method includes receiving pilot monitoring data sensed by cockpit-based physiological sensors.

In some embodiments, the method includes determining pre-flight the necessary authority level for approval of the subsequent flight plan, based on the pre-flight risk assessment.

In some embodiments, the pilot profile includes a baseline performance level of the pilot, and the method includes adding indicators of the effect of the duration of the flight plan, and/or of a particular component flight operation of the flight plan, on the pilot's baseline performance.

In some embodiments, the method includes providing a rest period recommendation for the pilot based on the post-flight assessment of the pilot's fatigue state (e.g., based on guidelines for defining and interpreting the pilot's current fatigue state).

This Summary is provided solely as an introduction to subject matter that is fully described in the Detailed Description and Drawings. The Summary should not be considered to describe essential features nor be used to determine the scope of the Claims. Moreover, it is to be understood that both the foregoing Summary and the following Detailed Description are example and explanatory only and are not necessarily restrictive of the subject matter claimed.

### BRIEF DESCRIPTION OF THE DRAWINGS

The detailed description is described with reference to the accompanying figures. The use of the same reference numbers in different instances in the description and the figures may indicate similar or identical items. Various embodiments or examples ("examples") of the present disclosure are disclosed in the following detailed description and the accompanying drawings. The drawings are not necessarily to scale. In general, operations of disclosed processes may be performed in an arbitrary order, unless otherwise provided in the claims. In the drawings:
FIG. 1 is a block diagram illustrating a system for assessing fulfillment of a mission plan by a pilot or crewmember both post-flight and pre-flight based on sensed physiological data according to example embodiments of this disclosure; and
FIG. 2 is a flow diagram illustrating a method for assessing mission fulfillment by a pilot or crewmember according to example embodiments of this disclosure.

### DETAILED DESCRIPTION

Before explaining one or more embodiments of the disclosure in detail, it is to be understood that the embodiments are not limited in their application to the details of construction and the arrangement of the components or steps or methodologies set forth in the following description or illustrated in the drawings. In the following detailed description of embodiments, numerous specific details may be set forth in order to provide a more thorough understanding of the disclosure. However, it will be apparent to one of ordinary skill in the art having the benefit of the instant disclosure that the embodiments disclosed herein may be practiced without some of these specific details. In other instances, well-known features may not be described in detail to avoid unnecessarily complicating the instant disclosure.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the disclosure in any way unless expressly stated to the contrary.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of "a" or "an" may be employed to describe elements and components of embodiments disclosed herein. This is done merely for convenience and "a" and "an" are intended to include "one" or "at least one," and the singular also includes the plural unless it is obvious that it is meant otherwise.

Finally, as used herein any reference to "one embodiment" or "some embodiments" means that a particular element, feature, structure, or characteristic described in connection with the embodiment is included in at least one embodiment disclosed herein. The appearances of the phrase "in some embodiments" in various places in the specification are not necessarily all referring to the same embodiment, and embodiments may include one or more of the features expressly described or inherently present herein, or any combination or sub-combination of two or more such features, along with any other features which may not necessarily be expressly described or inherently present in the instant disclosure.

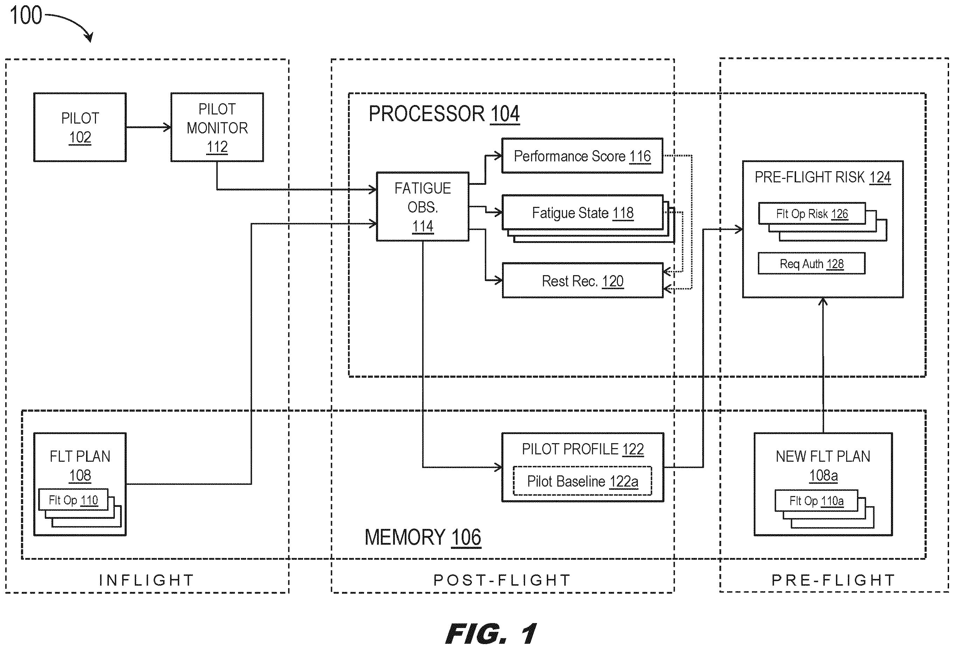

Referring now to FIG. 1, a system 100 for assessing fatigue in a pilot 102 or crewmember is shown. The system 100 may include one or more processors 104 and memory (e.g., data storage) 106.

Broadly speaking, the system 100 may utilize monitoring technology configured to monitor physiological states of a pilot 102 or crewmember inflight throughout the execution or fulfillment of a flight plan 108 (e.g., mission plan). When the flight plan 108 is fulfilled by the pilot 102, a current fatigue state of the pilot may be determined based on physiological data sensed inflight by pilot monitors as well as subsequent observations made by the system as to the fatigue level of the pilot at particular points associated with specific flight operations. Based on the current fatigue state of the pilot 102, the pilot's commander may more accurately determine whether additional rest is required in the short term.

Furthermore, fatigue observations based on the sensed physiological data may be added to a comprehensive profile specific to the pilot 102 and capable of assessing the effect of specific flight operations on the pilot's individual baseline performance level and fatigue tolerance. When the pilot 102 is assigned a subsequent mission, the system may provide the commander with an evidence-based pre-flight assessment of the risk involved in the fulfillment of that mission by that pilot, based on their known tolerance levels.

In embodiments, the pilot 102 may execute a flight plan 108 (e.g., mission plan) stored or storable within the memory 106. For example, the flight plan 108 may have a projected duration and may include a set or sequence of specific flight operations 110, e.g., IMC/IFR operations, visual meteorological conditions (VMC)/visual flight rules (VFR) operations, nighttime operations, operations under NVG, high-temperature operations. Throughout the execution of the flight plan, the pilot 102 may be monitored to assess performance, cognitive workload, awareness, and/or fatigue. For example, pilot monitoring systems 112 may include eye tracking systems (for observing eye positions, orientations, and ocular activity, e.g., blink rate/frequency, saccadic movements, gaze targets), functional near-infrared spectroscopy (fNIRS; e.g., brain imaging via measurement of blood oxygenation), and other physiological sensors capable of measuring parameters relevant to the pilot's alertness, fatigue level, and/or cognitive well-being at any point inflight. In embodiments, a particular time window inflight may be associated with one or more flight operations 110 under execution by the pilot 102 at that time. For example, each flight operation 110 may be associated with a projected duration relative to the duration of the flight plan 108, e.g., 0.8 hours simulated IMC, 1.2 hours daytime VFR, 0.5 hr. nighttime VFR (of a 12-hour mission duration). In some embodiments, the pilot 102 may manually provide this information to pilot monitoring systems 112; alternatively, the flight plan 108 stored to memory 106 may provide this information to the pilot monitoring systems and to the fatigue assessment system 100.

In embodiments, when the flight plan 108 is completed, the system 100 may receive any physiological data sensed inflight by the pilot monitoring systems 112. For example, the pilot monitoring systems 112 may correlate any physiological states observed with respect to the pilot 102 with any flight operations 110 currently under execution at the time of the observation.

In embodiments, the system 100 may perform postflight fatigue observations 114 based on the received sensed physiological data. For example, each postflight fatigue observation 114 may include a performance score 116 quantifying the overall performance of the pilot 102 in fulfillment of the flight plan 108. Further, each postflight fatigue observation 114 may include one or more assessments of the general fatigue state 118 of the pilot 102, currently and/or at specific times within the duration of the flight plan 108, e.g., times correlating to specific flight operations 110 underway at those times. In some embodiments, each assessment of fatigue state 118 may characterize the fatigue level of the pilot 102 broadly or generally, e.g., green=nominal, yellow=moderately fatigued, red=critical. In some embodiments, the assessment of fatigue state 118 may attempt to more precisely characterize the fatigue level of the pilot 102 at a particular time or corresponding to a particular flight operation 110, e.g., on a 20-point or even 100-point scale. For example, each assessment of fatigue state 118 may indicate any flight operations 110 ongoing at that time, any flight operations previously executed within the flight plan 108, and the duration of the flight plan executed at the time of the assessment. It may be noted, for example, that each flight operation 110 may be associated with a required level of cognitive workload, but that the precise level of cognitive workload associated with a particular flight operation may vary from pilot to pilot. For example, the pilot 102 may be able to fly for 2 hours under daytime VMC conditions before reaching a particular fatigue level, but may reach that same fatigue level after only one hour of flight under nighttime IMC conditions. Further, the cognitive effect of multiple varied flight operations 110 executed throughout the fulfillment of a flight plan 108 may be cumulative as the duration of the flight plan extends.

In some embodiments, the postflight fatigue observation 114 may further include rest period recommendations 120 based on a current performance score 116 or assessment of fatigue state 118 for the pilot 102. Further, when supervising authorities define how the assessment of fatigue state 118 will characterize the pilot's fatigue level (e.g., with what level of granularity, from a hierarchy of a few very broad categories to a more specific numeric scale), said authorities may further define recommended responses to a given assessment of fatigue state or category thereof, e.g., specific to a particular pilot 102 as opposed to standardized tables based on estimated fatigue levels. For example, under a conventional approach the pilot 102 may be recommended to fly for 7 hours before reaching the end of their duty day. Accordingly, any pilot 102 having concluded a 5-hour flight operation may be uniformly recommended for 2 further hours of subsequent flight operations 110a within a duty day before reaching their recommended safe limit, based on an estimated uniform fatigue level. In embodiments, however, the system may perform a post-flight assessment of fatigue state 118 for the pilot 102 (based on part on fatigue observations 114 derived from inflight physiological monitoring data) and, based on organizational guidelines for interpreting the current fatigue state of the pilot (e.g., as well as the flight performance score 116), may perform a more specialized rest recommendation 120 with respect to additional flight operations 110a by the pilot. For example, the specialized rest recommendation 120 may instead recommend more or less than 2 further hours of flight operations 110a (e.g., before the subsequent performance score 116 and/or assessment of fatigue state 118 of the pilot 102 is adversely affected beyond acceptable levels.

In embodiments, the system may add postflight fatigue observations 114 and/or sensed physiological data collected in fulfillment of the flight plan 108 to a pilot profile 122 specific to the pilot 102. For example, the pilot 102 may execute a first flight plan 108 comprising a first set of flight operations 110 as described above. In embodiments, based on physiological monitoring of the pilot 102 throughout the execution of this first flight plan 108, (e.g., in addition to any performance scores 116 and/or assessments of fatigue state 118 associated with the first flight plan or with specific flight operations 110 thereof), the system 100 may establish a baseline performance level 122a for the pilot. For example, as the pilot 102 executes subsequent flight plans 108a and component flight operations 110a, and as further physiological data is collected and further postflight fatigue observations 114 made, the baseline performance level 122a may be updated. Further, as the pilot 102 gains experience and the pilot profile 122 develops, the system 100 may learn how aspects of each flight plan 108 affect the fatigue level of the pilot. For example, the pilot profile 122 may reflect more precisely the effect on baseline performance level 122a of a flight plan 108 having a longer than average duration, or of a particular flight operation 110. For example, flight operations 110, 110a observed over time (e.g., over multiple flight plans 108, 108a and multiple hours dedicated to the particular flight operation) to present a higher cognitive workload to the pilot 102 may be scheduled earlier in a flight plan if possible, to minimize the risk of a significant adverse effect late in the flight plan of such a demanding flight operation.

In embodiments, by tracking the effect on baseline performance 122a of a variety of individual component flight operations 110 as well as the duration of a flight plan 108 generally, the system 100 may perform pre-flight risk assessments 124 when the pilot 102 is presented with a subsequent flight plan 108a associated with a subsequent or new set of flight operations 110a. For example, the subsequent flight plan 108a and its set of component flight operations 110a may be implemented similarly to the flight plan 108 and flight operations 110, but for a different duration or in different combinations. In embodiments, based on information provided by the pilot profile 122 with respect to the effect on baseline performance 122a of the duration of the flight plan 108a, of each component flight operation 110a thereof, or of the arrangement or sequencing of the flight operations 110a within the flight plan 108a, the system 100 may provide a general pre-flight risk assessment 122 associated with the capacity of the pilot 102 to execute the flight plan 108a (and, e.g., how the assessed fatigue state 118 of the pilot may evolve over the course of the flight plan 108a). Further, the general pre-flight risk assessment 124 may include more specific flight operation risk assessments 126 associated with the effect of a particular flight operation 110a on the fatigue state (e.g., on a subsequent assessment of fatigue state 118 based on the execution of said flight operation) of the pilot 102.

In some embodiments, the general pre-flight risk assessment 124 may include a required authority level 128 for authorization to assign the flight plan 108a to the pilot 102. For example, pre-flight risk assessments 124 based on standardized estimates of pilot fatigue levels may require approval of the associated flight plan 108a at a level of authority commensurate with the assessed level of risk: lower levels of risk may require approval of the flight plan by a company commander while higher levels may require approval at the battalion, brigade, or even division level. In embodiments, individualized assessments of fatigue state 118 (e.g., and/or historical fatigue levels as reflected by an individualized pilot profile 122) may sufficiently increase confidence levels associated with a particular pilot 102 as to allow approval of subsequent flight plans 108a and/or flight operations 110a by said pilot at lower levels of authority (e.g., from division-level or brigade-level down to battalion-level).

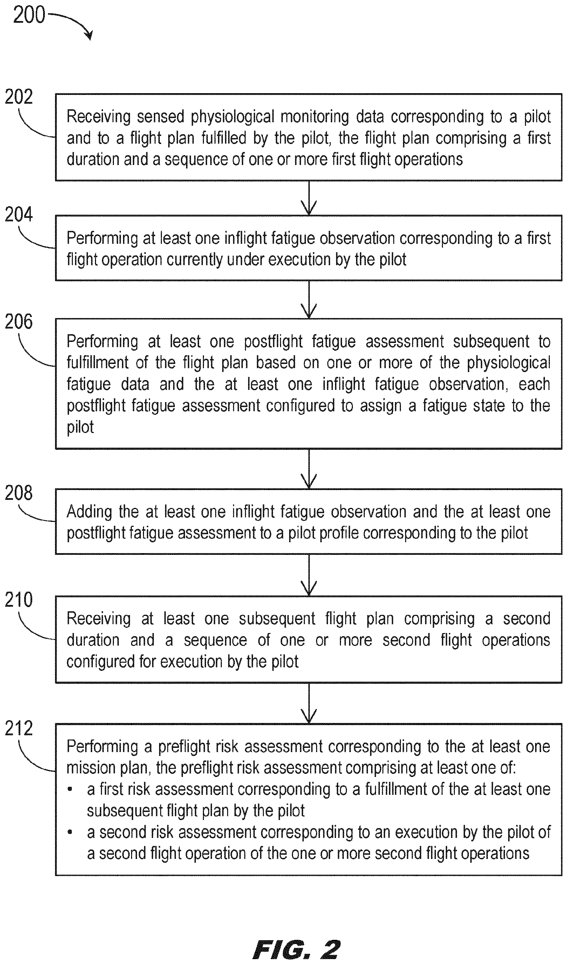

Referring to FIG. 2, the method 200 may be implemented by the system 100 and may include the following steps.

At a step 202, the system receives pilot monitoring data based on monitoring a pilot executing a particular flight plan or mission, the flight plan having a duration including a set or sequence of component flight operations. For example, the pilot monitoring data may be collected by eye trackers and/or other cockpit-based physiological sensors capable of tracking parameters relevant to the alertness, fatigue level, cognitive workload, and/or general capacity of the pilot. The pilot monitoring data may correlate component flight operations with the flight plan, or the pilot may manually correlate, e.g., by indicating component flight operations as they commence.

At a step 204, the system performs inflight fatigue observations corresponding to particular observation times and/or flight operations underway at the time of observation. For example, the system analyzes the received pilot monitoring data to assess the fatigue level of the pilot at particular points inflight, e.g., where a particular flight operation may be underway, may be about to commence, or may have just been completed.

At a step 206, the system performs a postflight fatigue assessment, e.g., after the pilot has completed fulfillment of the flight plan or mission. For example, the postflight fatigue assessment evaluates the current fatigue level of the pilot, e.g., upon completion of or subsequent to completing the flight plan. The postflight fatigue assessment may include additional assessments of fatigue state at points inflight, e.g., based on inflight fatigue observations and/or raw sensed physiological data. In some embodiments, the postflight fatigue assessment includes rest period and/or duty day restriction recommendations.

At a step 208, the inflight fatigue observations and postflight fatigue assessment are added to an individualized pilot profile. For example, each pilot profile may establish a baseline performance level for a particular pilot (e.g., based on one or more flight plans executed by that pilot and monitoring data collected throughout its execution). As the pilot executes additional flight plans, and logs additional hours completing component flight operations, the pilot profile tracks the effect of flight plan durations, of individual component flight operations, and of sequences of flight operations on the pilot's baseline performance level.

At a step 210, the system receives a new flight plan or mission for execution by the pilot, the new flight plan including a new set or sequence of component flight operations.

At a step 212, the system performs a preflight risk assessment based on the new flight plan and the pilot profile. For example, the preflight risk assessment may predict a general risk level associated with the capacity of the pilot to successfully fulfill or execute the flight plan, e.g., based on the likely effect of the new flight plan (e.g., duration of, component flight operations of) on the pilot's baseline performance level. In embodiments, a flight plan associated with a higher risk level for a particular pilot may accordingly require a higher level of authority to authorize assigning the flight plan to that pilot. The preflight risk assessment may additionally assess a risk level associated with a particular component flight operation (e.g., based on the cognitive workload associated with the flight operation and/or the sequencing of the flight operation within the duration of the flight plan).

### CONCLUSION

It is to be understood that embodiments of the methods disclosed herein may include one or more of the steps described herein. Further, such steps may be carried out in any desired order and two or more of the steps may be carried out simultaneously with one another. Two or more of the steps disclosed herein may be combined in a single step, and in some embodiments, one or more of the steps may be carried out as two or more sub-steps. Further, other steps or sub-steps may be carried in addition to, or as substitutes to one or more of the steps disclosed herein.

Although inventive concepts have been described with reference to the embodiments illustrated in the attached drawing figures, equivalents may be employed and substitutions made herein without departing from the scope of the claims. Components illustrated and described herein are merely examples of a system/device and components that may be used to implement embodiments of the inventive concepts and may be replaced with other devices and components without departing from the scope of the claims. Furthermore, any dimensions, degrees, and/or numerical ranges provided herein are to be understood as non-limiting examples unless otherwise specified in the claims.

## Claims

1. A system for assessing the fulfillment of a mission plan by a pilot, the system comprising:
a memory (106) configured for storage of:
at least one flight plan, each flight plan comprising a duration and a sequence of one or more flight operations;
and
processor-executable encoded instructions;
and
one or more processors (104) configurable by the encoded instructions for:
receiving physiological monitoring data corresponding to a pilot and a first flight plan fulfilled by the pilot, the physiological monitoring data sensed by at least one aircraft-based sensor;
performing, based on the received physiological monitoring data and the associated first flight plan, at least one inflight fatigue observation corresponding to at least one flight operation of the first flight plan;
performing, based on one or more of the received physiological monitoring data and the at least one inflight fatigue observation and subsequent to fulfillment of the first flight plan, at least one postflight fatigue assessment configured to assign a current fatigue state to the pilot;
adding the at least one postflight fatigue assessment and the at least one inflight fatigue observation to a pilot profile corresponding to the pilot;
receiving from the memory (106) at least one second flight plan for fulfillment by the pilot;
and
performing a preflight risk assessment corresponding to the at least one second flight plan, the preflight risk assessment comprising at least one of:
a first risk assessment corresponding to a fulfillment of the at least one second flight plan by the pilot;
and
a second risk assessment corresponding to an execution by the pilot of at least one flight operation of the at least one second flight plan.

2. The system of Claim 1, wherein the physiological monitoring data includes eye tracking data.

3. The system of Claim 1, wherein the physiological monitoring data includes monitoring data captured by at least one cockpit-based physiological sensor.

4. The system of any preceding Claim, wherein the first risk assessment includes an authority level necessary to authorize fulfillment of the at least one second flight plan by the pilot.

5. The system of any preceding Claim, wherein the pilot profile includes at least one of:
a baseline performance level of the pilot;
a first performance level indicative of an effect of the duration of the first flight plan on the baseline performance level;
or
a second performance level indicative of an effect on the baseline performance level of at least one flight operation of the first flight plan.

6. The system of any preceding Claim, wherein the at least one postflight fatigue assessment includes at least one rest period recommendation based on the current fatigue state.

7. A computer-assisted method for assessing a fulfillment of a mission plan by a pilot, the method comprising:
receiving (202) sensed physiological monitoring data corresponding to a pilot and to a flight plan fulfilled by the pilot, the flight plan comprising a first duration and a sequence of one or more first flight operations;
performing (204) at least one inflight fatigue observation corresponding to a first flight operation currently under execution by the pilot;
performing (206) at least one postflight fatigue assessment subsequent to fulfillment of the flight plan based on one or more of the physiological monitoring data and the at least one inflight fatigue observation, each postflight fatigue assessment configured to assign a fatigue state to the pilot;
adding (208) the at least one inflight fatigue observation and the at least one postflight fatigue assessment to a pilot profile corresponding to the pilot;
receiving (210) at least one subsequent flight plan comprising a second duration and a sequence of one or more second flight operations configured for execution by the pilot;
and
performing (212) a preflight risk assessment corresponding to the at least one subsequent flight plan, the preflight risk assessment comprising at least one of:
a first risk assessment corresponding to a fulfillment of the at least one subsequent flight plan by the pilot;
and
a second risk assessment corresponding to an execution by the pilot of a second flight operation of the one or more second flight operations.

8. The method of Claim 7, wherein the physiological monitoring data includes eye tracking data.

9. The method of Claim 7, wherein the physiological monitoring data includes monitoring data captured by at least one cockpit-based physiological sensor.

10. The method of Claim 7, 8 or 9, wherein the first risk assessment includes an authority level necessary to authorize fulfillment of the at least one subsequent flight plan by the pilot.

11. The method of any of Claims 7 to 10, wherein the pilot profile includes at least one of:
a baseline performance level of the pilot;
a first performance level indicative of an effect of the first duration on the baseline performance level;
or
a second performance level indicative of an effect of at least one first flight operation on the baseline performance level.

12. The method of any of Claims 7 to 10, wherein the at least one postflight fatigue assessment includes at least one rest period recommendation based on the current fatigue state.
